# EUROPEAN PATENT SPECIFICATION

(11) **EP 1 472 991 B1**
(45) Date of publication and mention of the grant of the patent: **11.06.2008**
(21) Application number: 04009876.6
(22) Date of filing: 26.04.2004
(51) Int. Cl.: A61F 2/06

(54) **Bifurcated stent with concentric body portions**
Verzweigter Stent mit konzentrischen Abschnitten
Stent avec bifurcation avec des corps principaux concentriques

(30) Priority: 25.04.2003 US 423840
(43) Date of publication of application: 03.11.2004
(73) Proprietor: Medtronic Vascular, Inc., Santa Rosa, CA 95403 (US)
(72) Inventor: Thornton, Ronan c/o Medtronic AVE Galway, Ballybrit Galway (IE)
(74) Representative: Hughes, Andrea Michelle

(56) References cited:
- EP-A- 1 293 178
- WO-A-00/27307
- US-A- 5 755 771

## Description

### TECHNICAL FIELD

This invention relates generally to biomedical stents. More specifically, the invention relates to a bifurcated stent comprising two stent frameworks, each framework including a leg portion and a body portion. The body portions are formed with segments and gaps that are concentrically aligned to provide minimal overlap of segments.

### BACKGROUND OF THE INVENTION

Stents are generally cylindrical-shaped devices that are radially expandable to hold open a segment of a vessel or other anatomical lumen after implantation into the lumen. Various types of stents are in use, including expandable and self-expanding stents. Expandable stents generally are conveyed to the area to be treated on balloon catheters or other expandable devices. For insertion, the stent is positioned in a compressed configuration along the delivery device, for example crimped onto a balloon that is folded or otherwise wrapped about a guide wire that is part of the delivery device. After the stent is positioned across the lesion, it is expanded by the delivery device, causing the diameter of the stent to expand. For a self-expanding stent, commonly a sheath is retracted, allowing expansion of the stent.

Stents are used in conjunction with balloon catheters in a variety of medical therapeutic applications, including intravascular angioplasty. For example, a balloon catheter device is inflated during percutaneous transluminal coronary angioplasty (PTCA) to dilate a stenotic blood vessel. The stenosis may be the result of a lesion such as a plaque or thrombus. When inflated, the pressurized balloon exerts a compressive force on the lesion, thereby increasing the inner diameter of the affected vessel. The increased interior vessel diameter facilitates improved blood flow.

Soon after the procedure, however, a significant proportion of treated vessels restenose. To prevent restenosis, a stent, constructed of a metal or polymer, is implanted within the vessel to maintain lumen size. The stent acts as a scaffold to support the lumen in an open position. Configurations of stents include a cylindrical tube defined by a solid wall, a mesh, interconnected stents, or like segments. Exemplary stents are disclosed in U.S. Patent No. 5,292,331 to Boneau, U.S. Patent No. 6,090,127 to Globerman, U.S. Patent No. 5,133,732 to Wiktor, U.S. Patent No. 4,739,762 to Palmaz, and U.S. Patent No. 5,421,955 to Lau.

Difficulties arise when the area requiring treatment is located near a bifurcation, the point at which a single vessel branches into two vessels. To effectively treat a vascular condition at a bifurcation, the stent must cover the entire affected area without obstructing blood flow in either adjoining vessel. This can be quite difficult to achieve.

Various conventional stenting techniques have been disclosed for treating bifurcations. One conventional bifurcation stenting technique includes first stenting the side-branch vessel and then the main vessel. Angle variations or limited visualization at the ostium (area at the opening) of the side-branch vessel may prevent accurate placement of the side-branch stent, resulting in the stent providing suboptimal coverage of the ostium or in the stent protruding into the main vessel and interfering with blood flow. The stent may, additionally, block access to portions of the adjoining vessel that require further intervention.

Another conventional technique involves first stenting the main vessel and then advancing a second stent through the wall of the main vessel stent and into the side-branch vessel, where the second stent is deployed. Disadvantages of this method include a risk of compressing the ostium of the side branch vessel when the main vessel stent is deployed, making insertion of a second stent difficult, if not impossible. Even when the side-branch vessel remains open, accurate positioning of a second stent through the wall of the first stent and into the side branch presents significant challenges and may result in undesirable overlapping of the stents.

Where the bifurcation forms a Y-shape, with the main vessel branching into two smaller vessels, conventional techniques have included placing three stents, one within the main vessel, and one within each of the smaller vessels. The problems discussed above may be present with this technique, as well.

WO 00/27307 discloses a variation of the so-called "Jo-Stent" in which a first stent is placed in the main vessel and a second stent is placed in the side branch such that the proximal parts of the two stents overlap in the main vessel.

Therefore it would be desirable to have a bifurcated stent and a system for treating a vascular condition that overcomes the aforementioned and other disadvantages.

### SUMMARY OF THE INVENTION

One aspect of the present invention is a bifurcated stent comprising:
a first stent framework including a first leg portion and a first body portion; and
a second stent framework including a second leg portion and a second body portion, wherein the first and second body portions are formed with segments and gaps; characterised in that the first and second body portions are concentrically aligned such that the segments of one body portion fill the gaps of the other body portion such that there is minimal or no overlap of the segments.

Another aspect of the present invention is a system for treating a vascular condition, comprising a catheter and a bifurcated stent as defined above.
A further aspect of the present invention is a method of manufacturing a bifurcated stent, comprising:
providing a first stent framework including a first leg portion and a first body portion having segments and gaps;
providing a second stent framework including a second leg portion and a second body portion having segments and gaps;
crimping each leg portion to a diameter less than that of the corresponding body portion; and
characterised by
concentrically aligning the first and second body portions such that the segments of one body portion fill the gaps of the other body portion such that there is minimal or no overlap of segments when the second body portion is concentrically aligned with the first body portion.

The aforementioned and other features and advantages of the invention will become further apparent from the following detailed description of the presently preferred embodiments, read in conjunction with the accompanying drawings. The detailed description and drawings are merely illustrative of the invention rather than limiting, the scope of the invention being defined by the appended claims.

### BRIEF DESCRIPTION OF THE DRAWINGS

FIG. 1 is an illustration of one embodiment of a first stent framework, in accordance with the present invention;
FIG. 2 is an illustration of one embodiment of a second stent framework, in accordance with the present invention;
FIG. 3 is an illustration of the stent framework of FIG. 1, showing the first stent framework after the first leg portion has been crimped to a diameter less than that of the first body portion;
FIG. 4 is an illustration of the stent framework of FIG. 2, showing the second stent framework after the second leg portion has been crimped to a diameter less than that of the second body portion;
FIG. 5 is an illustration of the first and second stent frameworks of FIG. 3 and FIG. 4 in position to be concentrically aligned;
FIG. 6 is an illustration of one embodiment of a bifurcated stent formed using the stent frameworks shown in FIG. 5, in accordance with the present invention;
FIG. 7 is an illustration of a system for treating a vascular condition, in accordance with the present invention; and .
FIG. 8 is a flow diagram of one embodiment of a method of manufacturing a bifurcated stent, in accordance with the present invention.

### DETAILED DESCRIPTION OF THE PRESENTLY PREFERRED EMBODIMENTS

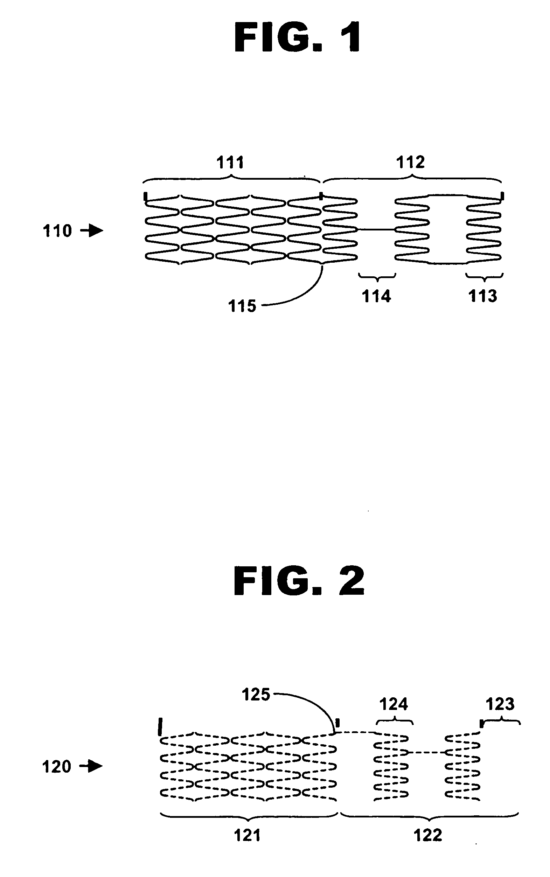

One aspect of the present invention is a bifurcated stent. One embodiment of the stent, in accordance with the present invention, is illustrated in FIGS 1 through 6. Like elements share like reference numbers in the figures.

The bifurcated stent of the present invention comprises a first stent framework 110 and a second stent framework 120. As shown in FIG. 1, first stent framework 110 includes a first leg portion 111 and a first body portion 112. The first body portion is formed with segments 113 and gaps 114. In this embodiment, first leg portion 111 is attached to first body portion 112 at a single point 115, which allows leg portion 111 to be crimped independently of body portion 112.

As seen in FIG. 2, second stent framework 120 includes a second leg portion 121 and a second body portion 122. The second body portion is formed with segments 124 and gaps 123. Second leg portion 121 is attached to second body portion 122 at a single point 125 to allow leg portion 121 to be crimped independently of body portion 122.

The stent frameworks may be made of a wide variety of medical implantable materials, such as stainless steel, nitinol, tantalum, ceramic, nickel, titanium, aluminum, polymeric materials, tantalum, MP35N, stainless steel, titanium ASTM F63-83 Grade 1, niobium, high carat gold K 19-22, or combinations of the above. The stent frameworks may be formed by cutting the patterns seen in FIG. 1 and FIG. 2 into the walls of tubular stent frameworks using a laser.

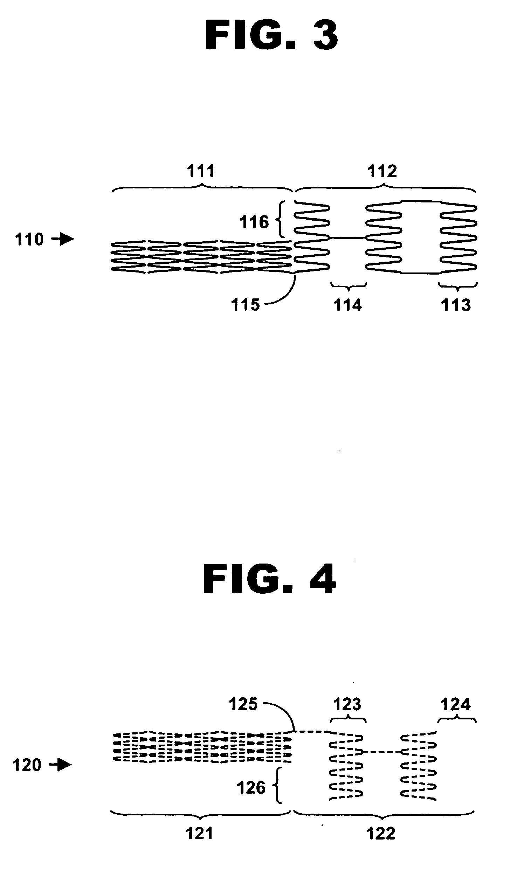

FIG. 3 shows first stent framework 110 with first leg portion 111 crimped to a diameter less than that of its corresponding body portion 112. In FIG. 4, second leg portion 121 of second stent framework 120 has also been crimped to a diameter less than that of its corresponding body portion 122. In both FIG. 3 and FIG. 4, the leg portions are shown crimped to a diameter slightly less than one-half that of the corresponding body portion. As can be seen, crimping of the leg portions leaves an opening 116 in first body portion 112 and an opening 126 in second body portion 122.

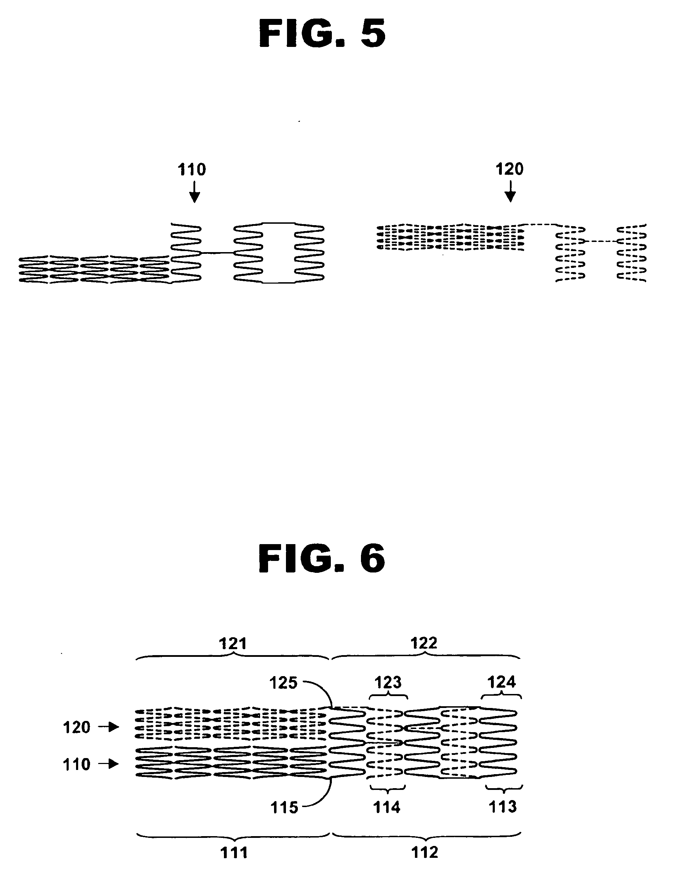

FIG. 5 illustrates first stent framework 110 and second stent framework 120 in position to be concentrically aligned. In FIG. 6, first stent framework 110 and second stent framework 120 have been assembled to form a bifurcated stent. The concentric alignment of first body portion 112 with second body portion 122 has been achieved by inserting second stent framework 120 into first stent framework 110, with second leg portion 121 passing through opening 116. Thus, second body portion 122 is within and concentrically aligned with first body portion 112. At least a distal end of body portion 122 may be crimped to facilitate inserting body portion 122 into body portion 112.

As can be seen, the segments 113 and gaps 114 of first body portion 112 and the segments 123 and gaps 124 of second body portion 122 align such that there is minimal overlap of the segments. Linkage points 115 and 125 are positioned to allow leg portions 111 and 121 to deflect one from the other.

A bifurcated stent in accordance with the present invention may include a therapeutic agent (not shown) disposed on at least a portion of the stent. The agent may be, for example, an antineoplastic agent, an antiproliferative agent, an antibiotic, an anti-inflammatory agent, combinations thereof, and the like.

The stent may further include a graft member (also not shown) such as a thin sleeve of polyester, expanded polytetrafluoroethylene (PTFE), or other appropriate material. The graft member may be positioned between the two body portions to act as a cushion between them, or it may be used to provide therapeutic benefits such as improved scaffolding or local delivery of a therapeutic agent in the vicinity of the bifurcation.

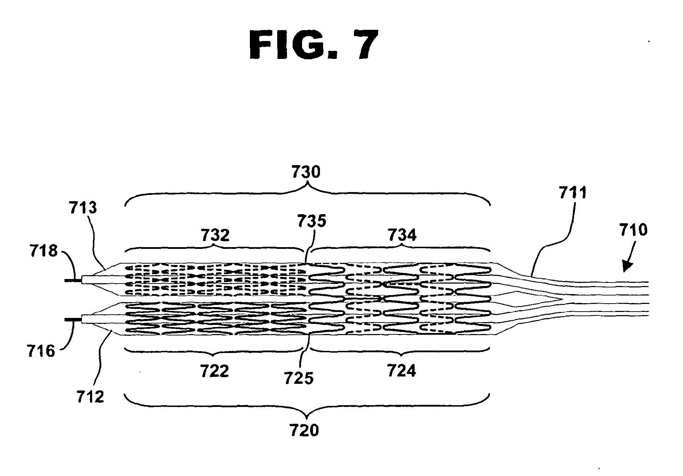

Another aspect of the present invention is a system for treating a vascular condition. One embodiment of the system, in accordance with the present invention, is illustrated in FIG. 7. The system comprises a catheter 710, a first stent framework 720, and a second stent framework 730.

Catheter 710 may be any catheter known in the art that is appropriate for delivering a stent to a treatment site within a vessel, for example a percutaneous transluminal coronary angioplasty (PTCA) balloon catheter.

First stent framework 720 and second stent framework 730 may be made of a wide variety of medical implantable materials, such as stainless steel, nitinol, tantalum, ceramic, nickel, titanium, aluminum, polymeric materials, tantalum, MP35N, stainless steel, titanium ASTM F63-83 Grade 1, niobium, high carat gold K 19-22, or combinations of the above. The stent frameworks may be formed by cutting patterns into tubular wall stent frameworks using a laser.

First stent framework 720 includes a first leg portion 722 and a first body portion 724. Second stent framework 730 includes a second leg portion 732 and a second body portion 734. Each leg portion is crimped to a diameter less than that of its corresponding body portion. The first body portion is linked to the first leg portion at 725 and the second body portion is linked to the second leg portion at 735, these points positioned to allow the leg portions to be crimped independently of the body portions. The linkage points are additionally positioned to allow the leg portions to deflect one from the other when the body portions are aligned, facilitating placement of each leg portion within a branch of a bifurcated vessel.

Body portions 724 and 734 are formed with segments and gaps. The two body portions are concentrically aligned such that there is minimal overlap of the segments. The concentric alignment may be achieved by inserting second stent framework 730 into first stent framework 720, with second leg portion 732 passing through an opening formed in first body portion 724 by the crimping of first leg portion 722. Thus, second body portion 734 is positioned within and concentrically aligned with first body portion 724. At least a distal end of second body portion 734 may be crimped to facilitate inserting body portion 734 into body portion 724.

In the embodiment shown in FIG. 7, catheter 710 includes a bifurcated balloon 711, which comprises a first balloon portion 712 and a second balloon portion 713. Bifurcated balloon 711 may be made of a suitable material such as polyethylene, polyethylene terephthalate (PET), nylon^{®}, pebax^{®}, or the like. The dimensions of balloon 711 may be selected based on the dimensions of the stent being delivered.

First leg portion 722 is operably coupled to first balloon portion 712. Second leg portion 732 is operably coupled to second balloon portion 713. The concentrically aligned first and second body portions 724 and 734 are operably coupled to both balloon portions, with the two balloon portions adjacent each other within the concentric body portions.

The system further includes a first guide wire 716 and a second guide wire 718. First guide wire 716 runs through a lumen within first balloon portion 712, while second guide wire 718 runs through a lumen within second balloon portion 713. The two guide wires aid in positioning leg portions 722 and 732 within the branches of a bifurcated vessel.

A system in accordance with the present invention may include a therapeutic agent (not shown) disposed on at least a portion of the system. The agent may be, for example, an antineoplastic agent, an antiproliferative agent, an antibiotic, an anti-inflammatory agent, or combinations thereof, and the like.

The system may further include a graft member (also not shown) such as a thin sleeve of polyester, expanded polytetrafluoroethylene (PTFE), or other appropriate material. The graft member may be positioned between the two body portions to act as a cushion between them, or it may be used to provide therapeutic benefits such as improved scaffolding or local delivery of a therapeutic agent in the vicinity of the bifurcation.

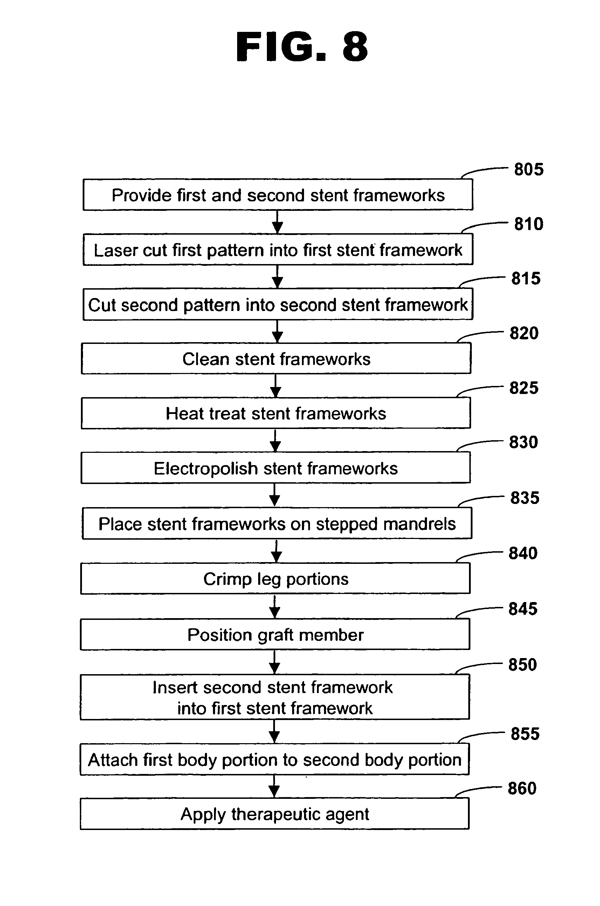

A further aspect of the present invention is a method of manufacturing a bifurcated stent. FIG. 8 shows a flow diagram of one embodiment of a method of manufacturing a bifurcated stent, in accordance with the present invention.

First and second stent frameworks are provided, each framework including a leg portion and a body portion (Block 805). Providing such a stent framework may include, for example, laser cutting a pattern through the wall of a tubular stent framework. A first pattern may be cut into the first stent framework (Block 810), while a second pattern may be cut into the second stent framework (Block 815). Both stent frameworks may be fully processed as for any laser cut stent. Thus, the stent frameworks may be cleaned (Block 820), heat treated (Block 815), and electropolished (Block 830) following laser cutting.

The pattern cut into the body portion of the first stent framework forms segments and gaps in the framework. The pattern cut into the body portion of the second stent framework also forms segments and gaps in the framework. These segments and gaps are positioned such that the segments of one body portion fill the gaps of the other body portion, thus providing minimal overlap of the segments when the two body portions are concentrically aligned. One skilled in the art will recognize that a wide variety of patterns may be devised to accomplish this and to provide other capabilities desired for the stent.

After laser cutting, each stent framework may be placed on a stepped mandrel for crimping (Block 835). An appropriate stepped mandrel would comprise two cylindrical portions, the first sized to fit snugly inside the stent framework, and the second having a diameter somewhat less than half that of the first mandrel portion. Each leg portion is then crimped down onto the mandrel portion having the smaller diameter (Block 840). This results in each leg portion having a diameter less than that of its corresponding body portion.

The patterns cut into the leg portions are designed not only to permit each leg portion to be crimped independently of its corresponding body portion, but also to enable the leg portions to deflect one from the other when the body portions are concentrically aligned. This ability to deflect facilitates placement of the leg portions within the branches of a bifurcated vessel. The leg and body portions may be linked at a single point or adjacent points on the stent framework, with the point(s) oriented appropriately on the mandrel during the crimping step.

A graft member may be positioned adjacent the second body portion prior to alignment of the two body portions (Block 845). The graft member may act as a cushion between the two body portions or may provide therapeutic benefits such as improved scaffolding and delivery of a therapeutic agent.

Concentric alignment of the body portions may be achieved by, for example, inserting the second stent framework into the first stent framework (Block 850), with the second leg portion passing through the opening formed in the first body portion when the first leg portion was crimped. The second body portion is thus positioned within and concentrically aligned with the first body portion, the segments of each body portion filling the gaps of the other body portion such that there is minimal overlap of the segments. At least a distal end of the second body portion may be crimped to facilitate insertion.

The body portion of the first stent framework may be attached to the body portion of the second stent framework using a method such as spot welding (Block 855). Such attachment may secure the stent frameworks one to the other, reducing or eliminating the risk of fretting between the two frameworks.

A therapeutic agent may be applied to provide local delivery of a therapeutic agent in the vicinity of the bifurcation (Block 860). The therapeutic agent may be applied to all or a portion of one or both stent frameworks. It may also be applied to all or a portion of the graft member. An application method such as dipping, spraying, pad printing, inkjet printing, rolling, painting, micro-spraying, wiping, electrostatic deposition, vapor deposition, epitaxial growth, and combinations thereof may be used. One skilled in the art will recognize that a therapeutic agent may be applied before, during, or after assembly of the stent.

## Claims

1. A bifurcated stent comprising:
a first stent framework (110) including a first leg portion (111) and a first body portion (112); and
a second stent framework (120) including a second leg portion (121) and a second body portion (122), wherein the first and second body portions are formed with segments (123) and gaps (124); **characterised in that** the first and second body portions are concentrically aligned such that the segments of one body portion fill the gaps of the other body portion such that there is minimal or no overlap of the segments.

2. The stent of claim 1 wherein each of the first stent framework and the second stent framework is formed by laser cutting a pattern into a tubular wall stent framework.

3. The stent of claim 1 wherein each leg portion is crimped to a diameter less than that of the corresponding body portion.

4. The stent of claim 1 wherein the first body portion is linked to the first leg portion and the second body portion is linked to the second leg portion at points positioned to allow the leg portions to be crimped independently of the body portions.

5. The stent of claim 4 wherein the points of linkage (115, 125) are positioned to allow the leg portions to deflect one from the other when the first and second body portions are concentrically aligned.

6. The stent of claim 1 wherein at least a distal end of one of the first body portion and the second body portion is crimped to facilitate concentric alignment of the body portions.

7. The stent of claim 1 further comprising:
a graft member positioned adjacent at least a portion of one of the first stent framework and the second stent framework.

8. The stent of claim 1 further comprising:
a therapeutic agent disposed on at least a portion of the stent.

9. A system for treating a vascular condition, comprising:
a catheter (710) and a bifurcated stent (720, 730) as claimed in any preceding claim;
the first and second stent frameworks being operably coupled to the catheter.

10. The system of claim 9 wherein the catheter includes a bifurcated balloon (711) having a first balloon portion (712) and a second balloon portion (713).

11. The system of claim 10 wherein the first leg portion is operably coupled to the first balloon portion, the second leg portion is operably coupled to the second balloon portion, and the concentrically aligned body portions are operably coupled to the first and second balloon portions together.

12. The system of claim 10 or 11 further comprising:
a first guide wire (716); and
a second guide wire (718).

13. A method of manufacturing a bifurcated stent, comprising:
providing a first stent framework (110) including a first leg portion (111) and a first body portion having segments (123) and gaps (126);
providing a second stent framework including a second leg portion and a second body portion (112) having segments and gaps;
crimping each leg portion to a diameter less than that of the corresponding body portion; and
**characterised by**
concentrically aligning the first and second body portions such that the segments of one body portion fill the gaps of the other body portion such that there is minimal or no overlap of segments when the second body portion is concentrically aligned with the first body portion.

14. The method of claim 13 wherein providing a first stent framework includes laser cutting a first pattern into a tubular wall stent framework.

15. The method of claim 14 wherein providing a first stent framework further includes performing steps selected from a group consisting of cleaning the stent framework, heating the stent framework, electropolishing the stent framework, and combinations thereof.

16. The method of claim 13, 14 or 15 wherein providing a second stent framework includes laser cutting a second pattern into a tubular wall stent framework.

17. The method of claim 16 wherein providing a second stent framework further includes performing steps selected from a group consisting of cleaning the stent framework, heating the stent framework, electropolishing the stent framework, and combinations thereof.

18. The method of claim 13 further comprising:
crimping at least a distal end of one of the first body portion and the second body portion to facilitate concentric alignment of the body portions.

19. The method of any of claims 13 to 18 further comprising:
positioning a graft member adjacent at least a portion of one of the first stent framework and the second stent framework.

20. The method of any of claims 13 to 19 further comprising:
applying a therapeutic agent to at least a portion of the stent.

21. The method of any of claims 13 to 20 further comprising:
attaching the body portion of the first stent framework to the body portion of the second stent framework.

## Patentansprüche

1. Gegabelter bzw. verzweigter Stent mit:
einem ersten Stentrahmenwerk bzw. -gerippe (110) mit einem ersten Beinabschnitt (111) und einen ersten Rumpfabschnitt (112); und
einem zweiten Stentgerippe (120) mit einem zweiten Beinabschnitt (121) und einen zweiten Rumpfabschnitt (122), bei dem die ersten und zweiten Rumpfabschnitte mit Segmenten (123) und Lücken (124) ausgebildet sind; **dadurch gekennzeichnet, dass** die ersten und zweiten Rumpfabschnitte konzentrisch ausgerichtet sind, so dass die Segmente eines Rumpfabschnittes die Lücken des anderen Rumpfabschnittes ausfüllen, so dass es eine minimale oder keine Überlappung der Segmente gibt.

2. Stent nach Anspruch 1, bei dem sowohl das erste Stentgerippe als auch das zweite Stentgerippe durch Laserschneiden eines Musters in ein Stentgerippe mit röhrenförmiger Wand ausgebildet ist.

3. Stent nach Anspruch 1, bei dem jeder Beinabschnitt auf einen Durchmesser gebördelt bzw. gecrimpt ist, der geringer ist als der des betreffenden Rumpfabschnittes.

4. Stent nach Anspruch 1, bei dem der erste Rumpfabschnitt mit dem ersten Beinabschnitt und der zweite Rumpfabschnitt mit dem zweiten Beinabschnitt an Punkten, die es erlauben, die Beinabschnitte unabhängig von den Körperabschnitten zu bördeln, verbunden sind.

5. Stent nach Anspruch 4, bei dem die Verknüpfungspunkte (115, 125) so angeordnet sind, dass sie den Beinabschnitten erlauben voneinander auszulenken, wenn die ersten und zweiten Rumpfabschnitte konzentrisch ausgerichtet sind.

6. Stent nach Anspruch 1, bei dem das distale Ende des ersten Rumpfabschnittes das distale Ende des zweiten Rumpfabschnittes, oder die distalen Enden beider Rumpfabschnitte gebördelt ist bzw. sind, um eine konzentrische Ausrichtung der Rumpfabschnitte zu erleichtern.

7. Stent nach Anspruch 1, ferner mit:
einem Transplantatelement, das neben einem Abschnitt des ersten Stentgerippes, des zweiten Stentgerippes, oder beider Stentgerippe angeordnet ist.

8. Stent nach Anspruch 1, ferner mit:
einem therapeutischen Wirkstoff, der wenigstens auf einem Abschnitt des Stents verteilt ist.

9. System zur Behandlung einer Gefäßkrankheit bzw. eines Gefäßzustands mit:
einem Katheter (710) und einem gegabelten Stent (720, 730) wie in einem der vorstehenden Ansprüche beansprucht;
wobei die ersten und die zweiten Stentgerippe operabel an den Katheter gekoppelt sind bzw. mit diesem in Wirkverbindung stehen.

10. System nach Anspruch 9, bei dem der Katheter einen gegabelten Ballon (711) mit einem ersten Ballonabschnitt (712) und einem zweiten Ballonabschnitt (713) beinhaltet.

11. System nach Anspruch 10, bei dem der erste Beinabschnitt operabel mit dem ersten Ballonabschnitt, der zweite Beinabschnitt operabel mit dem zweiten Ballonabschnitt und die konzentrisch ausgerichteten Rumpfabschnitte operabel mit den ersten und zweiten Ballonabschnitten zusammen verbunden sind bzw. in Wirkverbindung stehen.

12. System nach Anspruch 10 oder 11, ferner mit:
einem ersten Führungsdraht (716); und
einem zweiten Führungsdraht (718).

13. Verfahren zur Herstellung eines gegabelten Stents, mit:
Bereitstellung eines ersten Stentgerippes (110) mit einem ersten Beinabschnitt (111) und einem ersten Rumpfabschnitt mit Segmenten (123) und Lücken (126);
Bereitstellung eines zweiten Stentgerippes mit einem zweiten Beinabschnitt und einem zweiten Rumpfabschnitt (112) mit Segmenten und Lücken;
Bördeln jedes Beinabschnitts auf einen Durchmesser, der geringer ist als der des betreffenden Rumpfabschnittes; und
**gekennzeichnet durch**
konzentrisches Ausrichten der ersten und zweiten Rumpfabschnitte, so dass die Segmente des einen Rumpfabschnittes die Lücken des anderen Rumpfabschnittes ausfüllen, so dass es wenig oder kein Überlappen der Segmente gibt, wenn der zweite Rumpfabschnitt konzentrisch mit dem ersten Rumpfabschnitt ausgerichtet wird.

14. Verfahren nach Anspruch 13, bei dem das Bereitstellen eines ersten Stentgerippes das Laserschneiden eines ersten Musters in ein Stentgerippe mit röhrenförmiger Wand beinhaltet.

15. Verfahren nach Anspruch 14, bei das Bereitstellen eines ersten Stentgerippes ferner Schritte umfasst, die aus einer Gruppe ausgewählt sind, die besteht aus dem Reinigen des Stentgerippes, dem Erhitzen des Stentgerippes, dem Elektropolieren des Stentgerippes, und Kombinationen hieraus.

16. Verfahren nach Anspruch 13, 14 oder 15, bei dem das Bereitstellen eines zweiten Stentgerippes das Laserschneiden eines zweiten Musters in ein Stentgerippe mit röhrenförmiger Wand beinhaltet.

17. Verfahren nach Anspruch 16, bei dem das Bereitstellen eines zweiten Stentgerippes ferner Schritte beinhaltet, die aus einer Gruppe ausgewählt sind, die besteht aus dem Reinigen des Stentgerippes, dem Erhitzen des Stentgerippes, dem Elektropolieren des Stentgerippes, und Kombinationen hieraus.

18. Verfahren nach Anspruch 13, ferner mit:
Bördeln eines distalen Endes des ersten Rumpfabschnittes, des zweiten Rumpfabschnittes oder beider Rumpfabschnitte, um die konzentrische Anordnung der Rumpfabschnitte zu erleichtern.

19. Verfahren nach einem der Ansprüche 13 bis 18, ferner mit:
Anordnen eines Transplantatelements neben einem Abschnitt des ersten Stentgerippes, des zweiten Stentgerippes oder beider Stentgerippe.

20. Verfahren nach einem der Ansprüche 13 bis 19, ferner mit:
Aufbringen eines therapeutischen Wirkstoffs auf wenigstens einen Abschnitt des Stents.

21. Verfahren nach einem der Ansprüche 13 bis 20, ferner mit:
Anfügen des Rumpfabschnitts des ersten Stentgerippes an den Rumpfabschnitt des zweiten Stentgerippes.

## Revendications

1. Endoprothèse ou stent bifurquée comportant:
une première ossature d'endoprothèse (110) incluant une première partie de jambe (111) et une première partie de corps (112) ; et
une seconde ossature d'endoprothèse (120) incluant une seconde partie de jambe (121) et une seconde partie de corps (122),
dans laquelle les première et seconde parties de corps sont formées en ayant des segments (123) et des espaces (124) ; **caractérisée en ce que** les première et seconde parties de corps sont alignées concentriquement de telle sorte que les segments d'une partie de corps remplissent les espaces de l'autre partie de corps de telle sorte qu'il y a un chevauchement minimal ou qu'il n'y aucun chevauchement des segments.

2. Endoprothèse selon la revendication 1 dans laquelle chacune des première ossature d'endoprothèse et seconde ossature d'endoprothèse est formée en découpant au laser un motif dans une ossature d'endoprothèse à paroi tubulaire.

3. Endoprothèse selon la revendication 1 dans laquelle chaque partie de jambe est sertie jusqu'à un diamètre inférieur à celui de la partie de corps correspondante.

4. Endoprothèse selon la revendication 1 dans laquelle la première partie de corps est reliée à la première partie de jambe et la seconde partie de corps est reliée à la seconde partie de jambe au niveau de points positionnés pour permettre de sertir les parties de branche indépendamment des parties de corps.

5. Endoprothèse selon la revendication 4 dans laquelle les points de liaison (115, 125) sont positionnés pour permettre aux parties de jambe de dévier l'une par rapport à l'autre lorsque les première et seconde parties de corps sont alignées concentriquement.

6. Endoprothèse selon la revendication 1 dans laquelle au moins une extrémité distale de l'une des première partie de corps et seconde partie de corps est sertie pour faciliter l'alignement concentrique des parties de corps.

7. Endoprothèse selon la revendication 1 comportant également:
un élément de greffon positionné adjacent à au moins une partie d'une ossature parmi les première ossature d'endoprothèse et seconde ossature d'endoprothèse.

8. Endoprothèse selon la revendication 1 comportant en outre :
un agent thérapeutique disposé sur au moins une partie de l'endoprothèse.

9. Système pour traiter un état vasculaire, comportant :
un cathéter (710) et une endoprothèse bifurquée (720, 730) comme revendiqué dans une revendication précédente quelconque ;
les première et seconde ossatures d'endoprothèse étant reliées au cathéter de manière opérationnelle.

10. Système selon la revendication 9 dans lequel le cathéter inclut un ballonnet bifurqué (711) ayant une première partie de ballonnet (712) et une seconde partie de ballonnet (713).

11. Système selon la revendication 10 dans lequel la première partie de jambe est reliée de manière opérationnelle à la première partie de ballonnet, la seconde partie de jambe est reliée de manière opérationnelle à la seconde partie de ballonnet, et les parties de corps concentriquement alignées sont reliées de manière opérationnelle aux première et seconde parties de ballonnet ensemble.

12. Système selon la revendication 10 ou 11 comportant également :
un premier fil-guide (716) ; et
un second fil-guide (718).

13. Procédé de fabrication d'une endoprothèse bifurquée, comportant les étapes consistant à :
fournir une première ossature d'endoprothèse (110) incluant une première partie de jambe (111) et une première partie de corps ayant des segments (123) et des espaces (126) ;
fournir une seconde ossature d'endoprothèse incluant une seconde partie de jambe et une seconde partie de corps (112) ayant des segments et des espaces ;
sertir chaque partie de branche jusqu'à un diamètre inférieur à celui de la partie de corps correspondante ; et **caractérisé par** l'étape consistant à
aligner concentriquement les première et seconde parties de corps de telle sorte que les segments d'une partie de corps remplissent les espaces de l'autre partie de corps de telle sorte qu'il y a un chevauchement minimal ou qu'il n'y a aucun chevauchement de segments lorsque la seconde partie de corps est concentriquement alignée avec la première partie de corps.

14. Procédé selon la revendication 13 dans lequel la fourniture d'une première ossature d'endoprothèse inclut la découpe au laser d'un premier motif dans une ossature d'endoprothèse à paroi tubulaire.

15. Procédé selon la revendication 14 dans lequel la fourniture d'une première ossature d'endoprothèse inclut également l'exécution d'étapes sélectionnées parmi un groupe consistant à nettoyer l'ossature d'endoprothèse, chauffer l'ossature d'endoprothèse, polir par voie électrolytique l'ossature d'endoprothèse, et leurs combinaisons.

16. Procédé selon la revendication 13, 14 ou 15 dans lequel la fourniture d'une seconde ossature d'endoprothèse inclut la découpe au laser d'un second motif dans une ossature d'endoprothèse à paroi tubulaire.

17. Procédé selon la revendication 16 dans lequel la fourniture d'une seconde ossature d'endoprothèse inclut également l'exécution d'étapes sélectionnées parmi un groupe consistant à nettoyer l'ossature d'endoprothèse, chauffer l'ossature d'endoprothèse, polir par voie électrolytique l'ossature d'endoprothèse, et leurs combinaisons.

18. Procédé selon la revendication 13 comportant également l'étape consistant à :
sertir au moins une extrémité distale d'une partie parmi les première partie de corps et seconde partie de corps pour faciliter l'alignement concentrique des parties de corps.

19. Procédé selon l'une quelconque des revendications 13 à 18 comportant en outre l'étape consistant à :
positionner un élément de greffon adjacent à au moins une partie d'une ossature parmi les première ossature d'endoprothèse et seconde ossature d'endoprothèse.

20. Procédé selon l'une quelconque des revendications 13 à 19 comportant de plus l'étape consistant à :
appliquer un agent thérapeutique sur au moins une partie de l'endoprothèse.

21. Procédé selon l'une quelconque des revendications 13 à 20 comportant également l'étape consistant à :
fixer la partie de corps de la première ossature d'endoprothèse sur la partie de corps de la seconde ossature d'endoprothèse.
